# EUROPEAN PATENT APPLICATION

(11) **EP 1 382 365 A1**
(43) Date of publication of application: **21.01.2004**
(21) Application number: 01970502.9
(22) Date of filing: 11.06.2001
(51) Int. Cl.: A61M 21/00, A61B 5/16, E04H 3/08, B63B 29/02

(54) **METHOD FOR CORRECTING THE PSYCHOLOGICAL CONDITION OF A PERSON AND A FLOATING CENTRE FOR CARRYING OUT SAID METHOD**

(30) Priority: 10.04.2001 UA 01042413
(71) Applicant: Karpina, Taisia Stepanovna, Odessa, 04050 (UA)
(72) Inventor: Karpina, Taisia Stepanovna, Odessa, 04050 (UA)
(74) Representative: Säfwenberg, Björn
(86) International application number: PCT/UA2001/000014
(87) International publication number: WO 2002/081017

(57) **Abstract**

The present invention relates to methods for correction of psychological conditions in individuals, as well as to passenger ships for carrying out such methods and may be used in psychological practice and in the fields of medicine and shipbuilding. The technical effect attained by the floating centre according to the present invention is restoration of mental equilibrium, self-reliance, improvement of social adaptation and reaching feelings of self-affirmation.

According to the invention, an individual is placed in a spatially isolated passenger cabin at a floating centre for continuous stay during an individually chosen number of days ensuring his/her visual and spatial isolation. Under these conditions an individual is subject to strict daily regime and tests. His/her diet is modified and controlled. At the same time, an environment for physiological improvement and intellectual development is created. A psycho-physiological condition of an individual is monitored remotely with the use of physiological sensors and locally by visual inspection, and the conditions are gradually changed thus increasing the degree of individual's acclimation.

The structure of the ship serves its purpose. The hull and erection are made as a single structure having the closed space isolated from the boards by longitudinal partitions, this space being divided into four isolated zones where the rows of cabins are situated. Above the overall top surface of the closed space, a glass-enclosed promenade deck is situated. Cabins are situated in each isolated zone on truncated decks. Between the cabins there is a corridor having the height equal to the sum of heights of all truncated decks with the cabins.

## Description

### 1. Field of the invention

The present invention relates to methods for correction of psychological conditions in individuals, as well as to passenger ships for carrying out such methods and may be used in psychological practice for training people who work under extreme conditions, in the fields of medicine and shipbuilding.

### 2. Prior art

Known are methods for correction of psychological conditions in individuals using positive influence of elevated pressure in the presence of oxygen, in particular the method of hyperbaric oxygenation (e.g., SU 1648461, 1991). These methods require special medical equipment and prior medical examination and may be used for individuals having physiological causes for depression, suppressed states or conversely, for nervous agitation and related states.

The closest prior art for the method of correction of a psychological condition according to the present invention is a method disclosed in SU 1680166, 1991. This method is used for the correction of conditions which cause an individual to suffer undue fatigue, unsociability, anxiety and phobia. According to this method, an individual is placed in an isolated chamber with a specific regime, in particular the hyperbaric regime. The hyperbaric pressure is decreased to simulate the height of several thousand meters. The patient's condition is monitored over the course of a specified number of days and the regime is gradually adjusted over this time. The simulated ceiling height is increased, which progressively improves the degree of the individual's acclimation. The benefits of this regime manifest in an increased capacity for work, decrease of phobic reactions and increase of vigour.

In view of the technical solution according to the invention, this method is disadvantageous because of the necessity of using complicated medical equipment and its restriction to be used only for people who suffer clinical symptoms of the above-mentioned conditions found in the course of prior medical examination which supposes subjection to hypoxia. This method may be initiated only by medical personnel and is directed to the achievement of a curative effect. At the same time, this method may be performed strictly according to medical recommendations, while a patient's initiative is not taken into account.

E.G. Frid describes passenger ships having several decks and cabins on each deck separated by a central longitudinal corridor ( 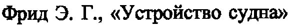 , J**I**.: 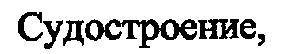 1990, cTp. 45/E.G. Frid. Structure of a ship. Leningrad: Sudostroyenie (shuipbuilding), 1990, page 45). This type of structure is comfortable and may be conveniently used in usual passenger and cruise ships.

The drawback of the above-mentioned structure, in view of the technical solution according to the invention, lies in limited ability to simultaneously monitor conditions of individuals staying in cabins situated on different levels separated by decks, as well as in inability to visually control cabins at the same time. Furthermore, cabins are primarily situated in an erection which does not allow to achieve the technical effect of the present invention.

The closest prior art for the technical solution according to the present invention is a passenger ship disclosed in Japanese patent application 3-46357, 1991, B 63 B 35/73. The ship includes an erection formed by two rows of cabins situated in pairs along both boards on several truncated decks so that one cabin in every pair of cabins opens to the inside of the ship and the other one opens to the outside. At the same time, any of the truncated decks with a corresponding row has an area smaller than that lying below. Therefore, every row is shaped like a truncated pyramid. There is also a free space between the rows of cabins shaped as a longitudinal corridor.

This construction of a passenger ship is disadvantageous because of the situation of cabin rows only in the erection, as well as in placing them in pairs so that their doors open to different sides of a corresponding row. Such construction does not provide isolation of passengers from the environment and does not afford to effect simultaneous visual control of the cabins.

### 3. Disclosure of the invention

One of the problems solved by the invention is developing a method for correction of a psychological condition of an individual which would simplify the process and raise its efficacy as compared to conventional methods due to the use of natural mechanisms of influence on the psycho-physiological condition of an individual along with psychological and training mechanisms.

Yet another problem solved by the present invention is developing a floating centre which would provide optimal conditions for carrying out said method.

One of the technical effects achieved by the invention may be providing possibilities to study, by methods used in psychology, conditions of individuals being under unusual and close to extreme conditions and develop techniques based on the conclusions made in this study for manipulating their psycho-physiological condition.

Furthermore, yet another technical effect may be providing a possibility to study individuals' conditions with the use of medical methods for the purpose of developing techniques for positive influence on an individual's psyche, as well as specialised systems of psychological and physiological training, etc.

The above problem is solved by the method for correction of a psychological condition of an individual according to which an individual is placed in a spatially isolated area with specific living conditions where his/her condition is monitored while the conditions are gradually changed and the degree of individual's acclimation is increased due to berthing him/her in said area for continuous stay during an individually chosen number of days ensuring his/her visual isolation, while spatial isolation is kept until the expiration of a pre-set term or before the person staying in this area gives a corresponding signal. Under these conditions an individual is subject to strict daily regime and tests. Furthermore, his/her diet is modified and controlled. At the same time, an environment for physiological improvement and intellectual development is created. Monitoring of a psycho -physiological condition of an individual is made remotely with the use of physiological sensors and locally by visual inspection.

Parameters used in tests are chosen individually. Background sounds and changes in temperature serve as the parameters of the tests. Spatial isolation is disturbed remotely or after the signal goes on, which is a bulb outside every cabin positioned to be seen by an observer.

The above-mentioned technical problem is also solved by a floating centre for carrying our said method, which is made as a passenger ship comprising a hull, erection, two rows of passenger cabins situated along both boards on several truncated decks and a free space shaped as a longitudinal corridor, provided between the rows of cabins, having the height equal to the sum of heights of all truncated decks, due to the hull and erection being made as a single structure having, inside of the rostrum and stern parts, closed spaces isolated from the boards by longitudinal partitions, each of these spaces being divided into lower and top isolated zones where the rows of cabins are situated, while above the overall top surface of these closed spaces a glass-enclosed promenade deck is situated.

The floating centre further includes a zone located between the closed spaces in the rostrum and stern parts, where facilities for improving physiological condition are to be situated, with a helipad being located over said zone.

Between the boards and longitudinal partitions promenade galleries are provided, while every truncated deck in the isolated zones of the closed spaces ends in a guard railing from the direction of the corridor. The cabins are opened remotely at a pre-set time or after the bulb signal outside every cabin goes on which is seen by an observer staying in the common corridor.

According to the method of the present invention, visual isolation from the environment is achieved due to making walls in the cabins without portholes. The cabins are illuminated by conventional illuminating lamps. In order to provide spatial isolation of the passengers, the doors of the cabins are made in such way that they may be opened only from the outside.

The method of the invention is meant for the correction of psychological conditions of individuals in need of restoring mental equilibrium, increase of self-reliance, improving social adaptation, or individuals aiming at hardening their will and self-affirmation by placing them, for a certain period of time, in an isolated area with no contact with other people, no luxuries and entertainment, and by creating additional conditions emphasising severity of circumstances while creating an environment necessary for training their body and mind. At the same time, every individual is constantly observed by psychologists, psychoanalysts, coaches and other specialists, and the living conditions for every individual are accordingly adjusted. This method is characterised in that it provides for constant interaction between a passenger and attendance crew and for co -ordination of the sequence of operations. Due to this method, any interested individual can make an objective assessment of his/her potential in out of the ordinary environment.

The floating centre for carrying out said method is made as a passenger ship on which passengers are berthed in cabins situated in two closed spaces occupying the majority of the ship below and above the waterline, these closed spaces being divided into two isolated zones - rostrum lower and top zones and, respectively, stern lower and top zones. In the centre, between the closed spaces, the ship has facilities meant to be used for various physical trainings by passengers. Above these facilities, there is a helipad suitable to provide safe landing of a helicopter in emergency cases. Cabins for hosting passengers, which are made with solid walls and no portholes, are situated in every isolated zone on truncated decks fenced by guard railings. Every cabin is opened remotely from the outside at a certain time or at the request of an individual staying in a cabin, following a bulb signal seen by an observer in the corridor. Between the rows of cabins and boards, promenade galleries for passengers are provided.

As is known from specialised literature in the psychology field, an individual willing to move away from life he/she leads in a society and restrict his/her living conditions to ascetic, severe, strict conditions maximum close to extreme conditions disclosed in the present specification, consciously strives to improve his/her personality or to harden his/her will, or go through unknown pungent sensations which are insufficient in the society, or just move away from everyday routine and stay in solitude. The objective of the attendance crew is to create an environment specific to every individual depending on what result, in other words what type of correction, he/she is seeking. Attaining a result of such stay will depend on the competence of the crew in fulfilling their tasks.

As in any work with people, one can not neglect the influence of human factors. Therefore, there may always be a possibility to stop one or another operation included in the complex of operations directed to psychological correction. In particular, if necessary physical activity is diversified and background sound and dietary regime are changed.

If necessary, promenades, medical examination and consultations with coaches are included in daily regimen. It is also possible to interrupt the operation of the centre before the normal time of the termination of the correction cycle. In this case passengers may be evacuated by helicopter.

In the inventor's opinion, the most suitable conditions for implementing the method of the invention would be to stay on the open sea or ocean. In this case, even without supplementary equipment and instruments, one can achieve complete isolation from the human habitat, while such conditions create the sensation of being face to face with this mighty element which can be withstood only provided than an individual completely regains self-control. This is a very powerful psychological factor which contributes to the present method the features which can not be developed in any other conditions on land, other than in the artificially modelled environment. However, in this case, artificial modelling will not be sufficient for an individual to become aware of his/her isolation and will limit a positive effect of individual's own initiative on application of the method which would significantly decrease its efficacy.

Furthermore, the conditions created by staying on the open sea improve efficacy of the method as they intensify the sensation of a severe environment and isolation from the mundane world. At the same time, the method uses natural background sounds and imitates the sound and action of roaring ocean waves which can hardly be tolerated even by a good sailor. Sailor's temper who, as is known, have strong will, who are courageous and possess high level of self-control may serve as one more evidence for the efficacy of the influence by the sea on those who wish to harden their will, to overcome psychological complexes or to improve self-control.

The structure of the floating centre according to the invention was specially developed for carrying out the method of the invention and was determined by the necessity to create specific environment for passengers staying at this centre. Isolation is provided due to creation of the closed space in the middle of the ship separated from the boards by longitudinal partitions, which meet the respective safety requirements for navigation.

The applied tests involve the use of natural factors accompanying a stay on the sea, in particular continuous natural background sound re-creating the sound and action of the sea or ocean with sea-winds. These factors may be modified.

At the request of passengers the route of the ship may be changed and thus climatic conditions in which passengers are staying may be changed. This causes natural change in temperatures. For example, in the first part of the trip the ship may navigate toward equator and the passengers will be dealing with heat. Afterwards, the ship may be turned in the opposite direction and move toward the North Pole. In a while, the passengers will be on the cold sea surrounded by ice-floes.

The cause-and-effect relation between the features of the present invention and the attained technical effect consists in that all conditions under which an individual is staying at the floating centre of the invention and subjected to the application of the present method and which constitute the features of the present technical solution are created on the natural basis. They may be modified as needed. There is no need for the use of special equipment and apparatus. This significantly simplifies the technique.

Furthermore, in this method an individual consults with specialists and chooses conditions for his stay himself, while in future he may interfere in the process of correction of these conditions. In other words, an individual stays under the impression that he is the one who manipulates the correction of his psychological condition. The conditions created by this method are so natural that an individual is not even aware of their influence. Such "self-correction" approach where an individual's will and wishes are involved, may significantly improve the efficacy of the influence on the psyche.

### 4. Brief description of the drawings

For the purpose of illustration of the floating centre according to the invention, the enclosed drawing includes three figures representing side, top and cross-section views of the ship.
Fig. 1 shows the side view of the ship;
Fig. 2 shows the top view of the ship;
Fig. 3 shows the cross-section view of the ship (A-A).

### 5. Embodiments of the invention

The method of the present invention includes the following sequence of operations.

An individual wishing to improve his/her psychological condition due to this method consults with specialists and chooses starting conditions for staying at the floating centre. These conditions are meant to be chosen on an individual basis to ensure that the most efficient result is achieved. They primarily depend on what a certain individual is aiming at, whether to improve his/her personality, harden his/her will, increase self-control level, or stay in solitude to be able to concentrate on his/her own thoughts in this out of the ordinary environment, or to go through extreme sensations, etc. These conditions may be chosen according to several different embodiments.

For example, if an individual is courageous enough and aims at hardening his/her will, testing his/her capabilities or devoting his/her time to physical improvement, it is possible to apply strict daily regime and diet, change temperature in the cabin, provide complete visual and spatial isolation, offer different kinds of physical training and ongoing medical supervision.

For an individual planning to increase self-control level or rid oneself of bad habits, a well-planned daily regime and diet may be recommended along with visual and spatial isolation and training directed to the improvement of body and mind, regular promenades and continuous control by the personnel.

When dealing with an hyper-sensitive individual in need of recovering his/her mental equilibrium, overcoming complexes and self-affirmation, the conditions of his/her stay must be more flexible and chosen so that they could be easily changed, if necessary. Examination must be performed by specialist on a regular and thorough basis.

Conditions of individuals staying in cabins will be monitored by experienced staff consisting of psychologists, psychoanalysts, coaches and other specialists who will monitor the individual remotely and locally through visual inspection. In the event of any discomfort or need of information or assistance, a staff member may be sumoned with the communication devices installed in cabins.

The duration of stay at the floating centre is chosen on an individual basis and depends on the goals pursued by the individual who decides to correct his/her psychological condition and recommendations of specialists.

Passengers may refuse the inspection by specialists and use their time at the floating centre for relaxation optionally using facilities for physical training and other recreation services offered by the centre.

The ship made as a floating centre is a single structure uniting the hull and erection.

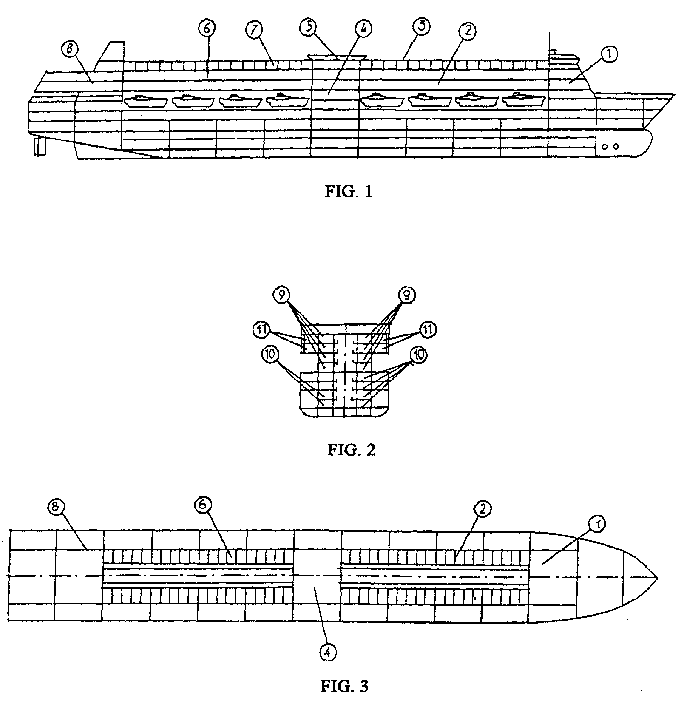

Figure 1 shows the side view of the ship. The rostrum includes the section (1) hosting living quarters and service rooms for the crew and personnel. The middle portion of the ship has a closed space isolated from the boards by longitudinal partitions and divided into four isolated zones where rows of cabins are situated. The rostrum block of cabins (2) includes lower and upper zones where cabins are situated, while over the upper surface of the closed space of the rostrum block of cabins (2) a glass-enclosed rostrum promenade deck (3) is situated.

The stern block is separated from the rostrum by yet another block of facilities (4) for the use by passengers for physical training. This block includes practice halls, weight rooms with exercise equipment, swimming-pools, etc. Above the block (4) there is a helipad (5) for the landing and taking-off of a helicopter.

The stem block (6) also includes lower and upper zones where cabins are situated and a promenade deck (7) situated above. The stem part has yet another block (8) of living quarters and service rooms for the crew and personnel.

Figure 2 shows cross-section view A-A where one can clearly see the arrangement of cabins in the rostrum (2) and stern (6) bocks. The cabins in the top zone (9) are placed on three truncated decks fenced by guard railings. Cabins in the lower zone (10) are placed on four decks three of which are truncated and end in guard railings. The top zone has promenade galleries (11) situated on two top decks under the promenade deck between the boards and longitudinal partitions.

Figure 3 shows top view of the ship. This image clearly represents general assembly of the ship, isolated zones (2) and (6), block of facilities (4) to be used by passengers for physical training, as well as blocks (1) and (8) of living quarters and service rooms for the crew and personnel.

Passengers continuously stay at the cabins in the top and lower zones (2) of the rostrum block and in the top and lower zones (6) of the stem block. The cabins provide visual isolation as no portholes are present. The doors of cabins open in the narrow corridor on the truncated deck are fenced by a guard railing. The doors are equipped with signal bulbs which may be activated from the inside. The doors open from the outside remotely at a pre-set time or following a signal from inside if a passenger wishes to see a member of the personnel, take an unplanned walk or get to any other place.

Staying in cabins, passengers follow a pre-set schedule developed by specialists based on their individual needs, physiological and psychological condition. Cabins are equipped with sound devices allowing for amplification of the sea-sounds, as well as thermostats which may either be set at a comfortable level or in the regime allowing to tighten the trial. The possibility of changing temperature regimes is also provisionally approved by specialists. The centre is also equipped with aeration devices used for supplying fresh air in cabins. Corridors are provided between the right and left sides of top and lower zones in the rostrum and stern blocks, which open view to every deck and, respectively, to the doors of all cabins on both sides. This allows the staff to see the signal bulb on the cabin doors. The same signals are received on a remote control desk situated is a special room. However, visual observance allows redundancy in the information received from a passenger and thus improve the operation of the specialists and other personnel.

Being on the ship, a passenger may chose his/her living conditions at any time. To this end he/she applies to a corresponding specialist and they correct certain parameters together. A passenger may, at any time, interrupt his/her stay on the ship and leave by helicopter taking off from the helipad (5).

Testing a passenger's condition made when the conditions of his stay at the centre are chosen, as well as during his stay when tests are made for the purpose of correcting passenger's living conditions and at the end of his/her stay at the centre in order to assess the results of his/her stay, show the efficacy of the method in its influence on psycho-physiological condition of an individual and allow to develop necessary recommendations. The test results may be used for different research of human physiology. They may also make their input in behavioural studies of individuals in close to extreme situations and in the development of recommendations for the choice of optimal conditions for hardening will and improving the physiological capabilities of a human.

## Claims

1. A method for correction of a psychological condition of an individual according to which an individual is placed in a spatially isolated area with specific living conditions where his/her condition is monitored while the conditions are gradually changed and the degree of individual's acclimation is increased, ***characterised in that*** he/she is berthed in said area for continuous stay during an individually chosen number of days ensuring his/her visual isolation, while spatial isolation is kept until the expiration of a pre-set term or before the person staying in this area gives a corresponding signal, and under these conditions an individual is subject to strict daily regime and tests, his/her diet is modified and controlled while an environment for physiological improvement and intellectual development is created, and monitoring of a psycho-physiological condition of an individual is made remotely with the use of physiological sensors and locally by visual inspection.

2. The method of claim 1, ***characterised in that*** parameters used in tests are chosen individually.

3. The method of claim 1 or 2, ***characterised in that*** background sounds and changes in temperature serve as the parameters of the tests.

4. The method of claim 1 to 3, ***characterised in that*** spatial isolation is disturbed remotely or after the signal goes on, which is a bulb outside every cabin positioned to be seen by an observer.

5. A floating centre made as a passenger ship comprising a hull, erection, two rows of passenger cabins situated along both boards on several truncated decks and a free space shaped as a longitudinal corridor, provided between the rows of cabins, having the height equal to the sum of heights of all truncated decks, ***characterised in that*** the hull and erection are made as a single structure having, inside of the rostrum and stem parts, closed spaces isolated from the boards by longitudinal partitions, each of these spaces being divided into lower and top isolated zones where the rows of cabins are situated, while above the overall top surface of these closed spaces a glass-enclosed promenade deck is situated.

6. The centre of claim 5, ***characterised in that*** it further includes a zone, where facilities for improving physiological condition are situated, over which a helipad is located.

7. The centre of claim 6, ***characterised in that*** the zone, where facilities for improving physiological condition are situated, is located between the closed spaces in the rostrum and stem parts.

8. The centre of claims 5 to 7, ***characterised in that*** between the boards and longitudinal partitions promenade galleries are provided.

9. The centre of claims 5 to 8, ***characterised in that*** every truncated deck in the isolated zones of the closed spaces ends in a guard railing from the direction of the corridor.

10. The centre according to claims 5 to 9, ***characterised in that*** the cabins are opened remotely at a pre-set time or after the bulb signal outside every cabin goes on which is seen by an observer staying in the common corridor.
